# EUROPEAN PATENT SPECIFICATION

(11) **EP 1 929 965 B1**
(45) Date of publication and mention of the grant of the patent: **27.01.2010**
(21) Application number: 06024996.8
(22) Date of filing: 04.12.2006
(51) Int. Cl.: A61B 17/28, A61B 17/22, A61B 17/115

(54) **A tissue clamp for transanal hemorrhoidopexy or hemorrhoidectomy**
Gewebeklemmvorrictung für transanale Hämorrhoidopexie oder Hämorrhoidektomie
Dispositif de fixation de tissu pour hémorroïdectomie ou hémorroïdopexie transanale

(43) Date of publication of application: 11.06.2008
(73) Proprietor: ETHICON ENDO-SURGERY, INC., Cincinnati, OH 45242-2839 (US)
(72) Inventor: Thompson, Brian James, Cincinnati, Ohio 45226 (US); Bowman, Heather, Cincinnati, Ohio 45226 (US); Pastorelli, Alessandro, 00136 Roma (IT)
(74) Representative: Leihkauf, Steffen Falk

(56) References cited:
- WO-A-01/91646
- WO-A-2006/027014
- US-A- 5 755 732
- US-A- 6 142 933
- US-A1- 2006 097 025

## Description

The invention relates in general to surgical instruments for the treatment of hemorrhoids and particularly to a tissue clamp for enabling a transanal hemorrhoidopexy or hemorrhoidectomy procedure to relocate internal hemorrhoids to their natural anatomical position and/or to remove a portion or the entirety of the internal hemorrhoids in patients suffering from mucosal prolapse. Hemorrhoids (piles) arise from congestion of internal and/or external venous plexuses around the anal canal and are characterized by a more or less pronounced tendency to prolapse temporarily or permanently outside of the anal canal. Symptoms associated with hemorrhoids include pain, bleeding, itching, mucus discharge and, in case of prolapsed tissue that cannot be manually replaced back inside of the anal canal, the prolapsed tissue is usually strangulated or thrombosed.

While in many cases hemorrhoidal disease can be treated by dietary modifications, topical medications and soaking in warm water, which temporarily reduce symptoms of pain and swelling, in a certain percentage of cases, surgical procedures are necessary to provide satisfactory, long-term relief.

These known surgical approaches imply traditionally the excision of the three major hemorrhoidal vessels (hemorrhoidectomy). A more recent approach, known as stapled hemorrhoidopexy or circumferential mucosectomy, reduces the prolapse of hemorrhoidal tissue by excising a band of the prolapsed anal mucosa membrane with the use of a circular stapling device.

Such a known circular stapling device comprises a staple fastening assembly with a proximal cartridge device and a distal anvil movable relative to the cartridge device. The cartridge device houses a staple- and knife-driving mechanism adapted to push two or more closed rows of staples and a circular knife out of a distal end surface of the cartridge device and towards the anvil. The anvil comprises a staple forming surface and an annular cutting block matching the staple rows and the circular knife of the cartridge device and is adapted to cooperate with the cartridge device for forming the ends of the staples exiting from the cartridge device and for excising the excess tissue by the circular knife. In order to position the band of prolapsed anal mucosa membrane between the cartridge device and the anvil of the circular stapler, it is necessary to place a purse-string suture around the entire anal circumference and to pull the prolapsed tissue by the suture inside the housing of the staple fastening assembly of the circular stapler. Although the use of the known surgical stapling instruments is beneficial and greatly facilitates the performance of a transanal hemorrhoidopexy or hemorrhoidectomy, it still involves some problems.

Due to the encumbrance of the cartridge device of the circular stapler, it is not possible for the surgeon to visually control the position and amount of tissue pulled into the instrument. This leads to an increased risk of rectal wall damages, e.g. when too much muscle tissue is drawn into the device and excised. Moreover, the structure of the known circular staplers render it difficult to gain access to the anal canal and the tissue to be excised may be too bulky to be incorporated into the housing of the stapling device.

Last but not least, the circular surgical staplers are rather complex instruments which combine and perform several functions or procedural steps of a hemorrhoidopexy, i.e. tissue positioning (clamping), tissue fixation (stapling) and tissue excision (cutting). While such a single device approach is certainly generally desirable, it entails comparatively high instrument costs and the impossibility of varying single steps of the surgical hemorrhoid treatment and of using the surgical techniques which are considered the most appropriate for the patient (e.g, tissue fixation by suturing instead of stapling or tissue excision by radiofrequency or ultrasonic energy instead of circular cutting).

An example of a known tissue clamp for transanal surgery is disclosed e.g. in US 6,142,933 A.

In view of the drawbacks of the prior art, the object of the invention is to provide a surgical instrument for performing transanal hemorrhoidopexy or hemorrhoidectomy which permits a good visual control of the position and amount of prolapsed tissue to be fixated and excised, in order to minimize the risk of rectal wall damages.

Within the general scope of the main object, it is a further aim of the present invention to provide a surgical instrument for performing transanal hemorrhoidopexy or hemorrhoidectomy having features such as to provide sufficient access to the prolapsed tissue by further surgical instruments, in order to enable the surgeon to freely chose and change surgical techniques to be applied to the tissue.

It is a yet further aim of the present invention to provide a surgical instrument for performing transanal hemorrhoidopexy or hemorrhoidectomy having features such that the outside dimension of the instrument doesn't limit the volume of prolapsed tissue to be fixated and excised.

It is a yet further aim of the present invention to provide a surgical instrument for performing transanal hemorrhoidopexy or hemorrhoidectomy having a simple and cost-effective structure compared with known circular staplers.

These and other objects are achieved by a surgical tissue clamp for holding a portion of prolapsed tissue in a transanal hemorrhoidopexy or hemorrhoidectomy according to claim 1. The dependent claims cover advantageous embodiments of the invention.

According to the invention, the tissue clamp comprises an elongate insertion shaft, a handle arranged at a proximal end of the insertion shaft and a clamping head arranged at a distal end of the insertion shaft. The clamping head comprises a proximal jaw and a distal jaw movable relative to the proximal jaw and configured to cooperate with the proximal jaw for clamping the prolapsed tissue in a substantially radial orientation with respect to a longitudinal axis of the clamping head. An actuating device is operatively connected with said proximal and distal jaws and adapted to approximate the jaws and to hold them in a tissue clamping configuration. The clamping head defines at least one proximal access aperture configured to proximally expose the clamped tissue, thereby allowing the clamped tissue to be visualized and, if necessary, to be reached by additional instruments while it is positioned, e.g. pulled into the clamping head, and clamped between the jaws. This makes it possible to obtain a precise positioning of the prolapsed tissue between the jaws of the clamp and, hence, a clamping line which adapts very well to the subsequent steps of the hemorrhoidopexy or hemorrhoidectomy, i.e. tissue fixation and excision. Moreover, the proximal access aperture exposes the tissue to be clamped not only proximally, but it allows at least part of the volume of prolapsed tissue to be pulled through or to fall through the access opening out of the clamping head during its positioning, so that the volume of tissue subject to treatment is not limited by the overall dimension of the clamp as is the case with the known circular staplers. This is particularly beneficial in patients with large confluent hemorrhoids. Once the prolapsed tissue has been clamped between the jaws, the latter remain locked in their clamping configuration and the tissue can now be easily reached and manipulated by other instruments, such as surgical suture, staples, adhesives or ultrasonic devices to fixate the tissue as well as surgical knifes, scalpels, scissors and ultrasonic or radiofrequency devices for excising the excess tissue volume.

The proximal jaw comprises a slender clamping ring or ring-segment (in the following referred to as proximal clamping ring) defining a curved tissue clamping line and being connected to the insertion shaft by a proximal spacing frame, said spacing frame and said ring or ring-segment defining said proximal access apertures. Thanks to the ring shape of the jaw, the clamping line can be well adapted to the tubular shape of the anal canal and the slenderness of the ring or ring-segment, particularly its small longitudinal (distal-proximal) extension enables the surgeon to comfortably reach and manipulate both the tissue radially outside and immediately adjacent to the ring shaped clamping line and the tissue volume held radially inside the clamping line. Preferably, the longitudinal thickness of the clamping ring is less than 1/10 of its circumference in case of a complete circular ring or less than π/5 of its radius in case of a ring segment. According to one aspect of the invention, the proximal spacing frame comprises preferably three slender radial spokes. The circumferential thickness of the spokes is smaller than the circumferential extension of the proximal access apertures defined between adjacent spokes, thereby maximizing the passage opening for the three distinct volumes of prolapsed mucosa membrane associated to the three major hemorrhoidal vessels.

According to a further aspect of the invention the proximal spacing frame comprises a substantially U-shaped frame having two longitudinal and approximately parallel opposite legs which are distally connected to the proximal clamping ring or ring-segment as well as a proximal base spoke which connects the U-shaped frame to the insertion shaft. In this way, the proximal access apertures defined by the proximal ring or ring-segment and the longitudinal legs of the proximal spacing frame expose the clamped tissue both proximally and laterally (radially). Moreover, the U-shaped frame provides a basket like spacer feature acting both radially and proximally, thereby providing space both for the tissue volume held radially inside the clamping line and for other surgical instruments which can be transanally inserted to manipulate the prolapsed tissue while it is held in position by the clamping head.

Like the proximal jaw, also the distal jaw comprises preferably a clamping ring or ring-segment (in the following referred to as distal clamping ring) complementary to the proximal clamping ring or ring segment of the proximal jaw and connected by means of a distal spacing frame, preferably by two or more radial spokes, to a pulling rod received in the insertion shaft and operatively connected to the actuating device of the tissue clamp.

According to a preferred embodiment, the insertion shaft is substantially straight and rigid and has a diameter which is less than the diameter of the clamping head. Preferably, the shaft diameter is less than half the diameter of the clamping head, yet more preferably, the shaft diameter is less than a third of the diameter of the clamping head. Of course, also curved insertion shafts are contemplated by the invention and might be advantageous adapt both to the way the surgeon uses his hands and to particular anatomic situations of the patient.

According to an aspect of the invention, the actuating mechanism comprises a manual actuating member preferably arranged at the handle so that it can be actuated extracorporeally, and a moving mechanism operatively connected to the manual actuating member and to the distal and proximal jaws and adapted to approximate (and preferably also to separate) the jaws in response to an actuating movement of the manual actuating member. Advantageously, a locking mechanism is also operatively connected to the distal and proximal jaws and configured to lock the jaws in their tissue clamping configuration, thereby setting both hands of the surgeon free and render them available to further manipulate the clamped, i.e. held and positioned tissue.

According to an advantageous development of the invention, the locking mechanism is arranged inside or at the insertion shaft and the handle itself is detachable from the insertion shaft on the proximal side of the locking mechanism. This makes it possible to remove the handle after the jaws have clamped the prolapsed tissue in order to gain a better and less obstructed transanal access to the tissue while the clamping configuration of the jaws is maintained.

In order to allow the locking mechanism to be accommodated inside the insertion shaft without substantially increasing the dimension of the latter, it proved advantageous to embody the actuating mechanism with a jackscrew which is rotatable but translationally locked with respect to the proximal jaw, a threaded connecting portion of the pulling rod of the distal jaw meshing said jackscrew, a rotating knob mounted on the handle and torsionally coupled with the jackscrew such that a rotation of the rotating knob causes the jackscrew to screw on the pulling rod thereby approximating or, viceversa, separating the jaws. In this way, the frictional resistance in the screw connection between the jackscrew and the pulling rod provides the above said jaw locking feature. This frictional locking capability is further increased by the longitudinal clamping force which causes a mutual pre-stress of the threads that can only be overcome by actively forcedly rotating the jack-screw.

According to an alternative embodiment, the manual actuating member comprises a trigger lever mounted on the handle and said moving mechanism comprises e.g. a pinion and rack gear coupled to the trigger lever and to the pulling rod of the distal jaw.

In order to even better visualize the prolapsed tissue during its positioning between the jaws and during the subsequent surgical treatment, according to an embodiment, the tissue clamp defines internally a longitudinal scope channel adapted to pass a scope through to the clamping head. The scope channel preferably extends substantially along the longitudinal axis of the tissue clamp and has a proximal inlet opening arranged in the handle and a distal outlet opening arranged in the insertion shaft proximally of the proximal and distal jaws and directly facing towards or through the proximal access aperture. In this embodiment, it is particularly advantageous to embody the actuation mechanism by hollow tubular members defining internally the scope channel. This embodiment allows for an extracorporeal visual control via a monitor connected to a scope which can be inserted through the scope channel up to the clamping head.

In connection with such a hollow tissue clamp, it is advantageous to give also the distal spacing frame a substantially U shaped or quadrangular frame having two longitudinal approximately parallel opposite legs distally connected to the distal clamping ring or ring-segment of the distal jaw and a proximal base spoke connecting said U-shaped or quadrangular frame to the pulling rod, thereby eliminating an axial pulling rod which would otherwise obstruct the view of the scope. The distal spacing frame is advantageously configured such that its longitudinal legs are close to, preferably overlapping the longitudinal legs of the proximal spacing frame in order not to obstruct the proximal access apertures defined by the latter.

These and other details and advantages of the present invention shall be made apparent from the accompanying drawings and the description thereof, which illustrate embodiments of the invention and, together with the general description of the invention given above, and the detailed description of the invention given below, serve to explain the principles of the present invention.
- Figure 1 is a distal lateral isometric view of a surgical tissue clamp according to a first embodiment of the invention;
- Figure 2 is a proximal view of the surgical tissue clamp of figure 1, applied transanally to a patient;
- Figures 3 to 6 illustrate steps of a hemorrhoidal treatment performed using the tissue clamp in figure 1;
- Figure 7 is a distal lateral isometric view of a surgical tissue clamp according to a second embodiment of the invention in an open configuration;
- Figure 8 is a distal lateral isometric view of the surgical tissue clamp in figure 7 in a closed configuration;
- Figure 9 is an enlarged isometric view of a detail in figure 7;
- Figure 10 is an isometric side view of an anal dilator which can be used together with the surgical tissue clamp according to the invention;
- Figure 11 is an exploded view of the surgical tissue clamp in figure 7;
- Figure 12 is a longitudinally sectioned view of the surgical tissue clamp in figure 7;
- Figure 13 is an enlarged view of a detail in figure 12;
- Figure 14 is a distal lateral isometric view of a surgical tissue clamp according to a third embodiment of the invention;
- Figure 15 is a distal lateral isometric view of a surgical tissue clamp according to a fourth embodiment of the invention;
- Figure 16 is the same view of figure 15 with the handle housing partially removed;
- Figure 17 is a proximal view of the surgical tissue clamp in figure 15.

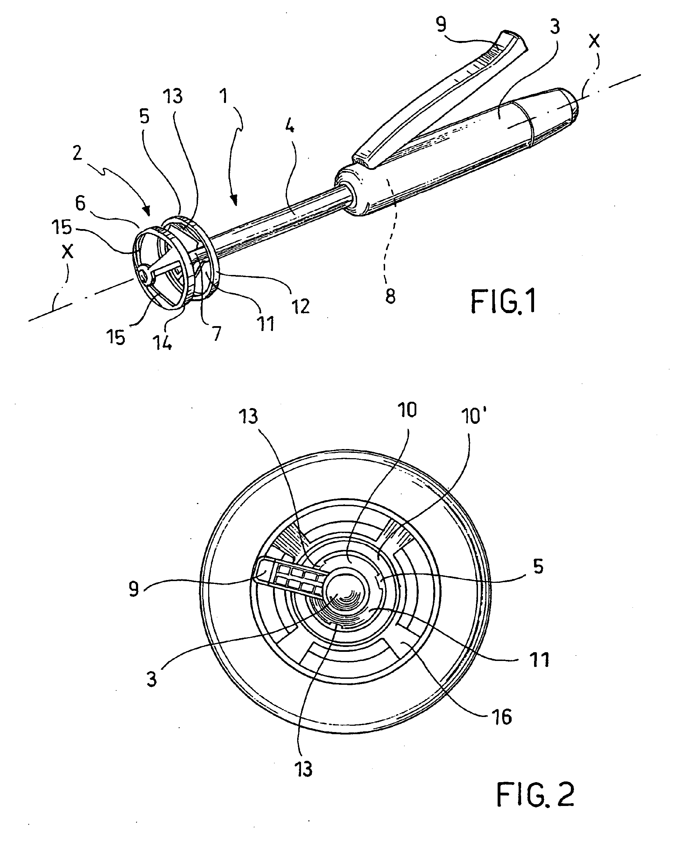

Turning to the figures, fig. 1 is an isometric overall view of a surgical tissue clamp 1 according to a first embodiment. The tissue clamp 1 comprises, in its distal end region, a clamping head 2 and, in its proximal end region, a handle 3. The handle 3 and the clamping head 2 are connected via a straight rigid insertion shaft 4 adapted to transanally insert and position the clamping head 2 by manually operating the handle 3 which is intended to remain outside the body of the patient.

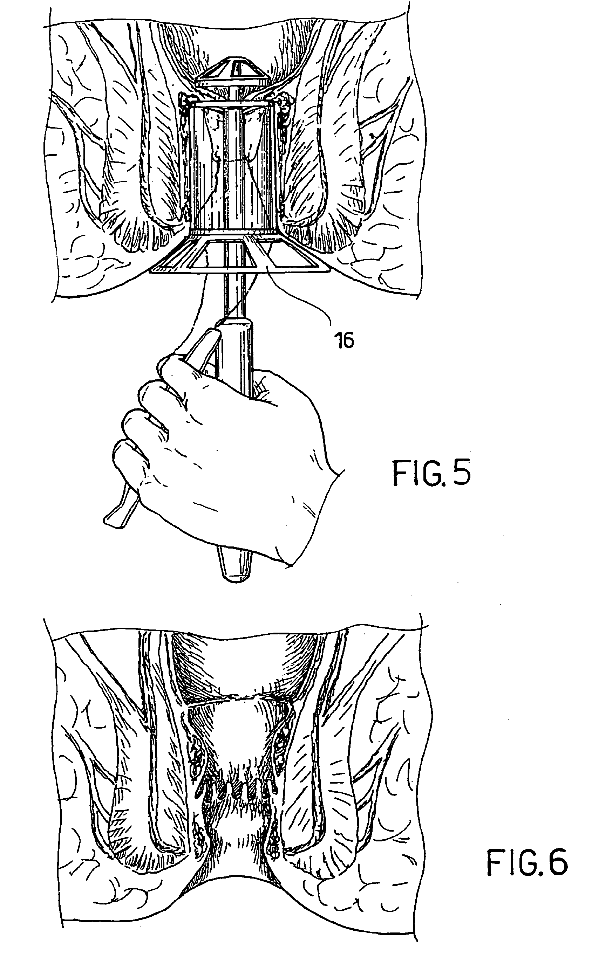

The clamping head 2 include a proximal jaw 5 rigidly connected to the distal end of the hollow tubular insertion shaft 4 and a distal jaw 6 connected to a distal end of a pulling rod 7 whose proximal end is slidingly housed inside the insertion shaft 4 such that the jaws can be translated relative to another and cooperate for clamping the prolapsed tissue in a substantially radial orientation with respect to a longitudinal axis X of the clamping head 2. The tissue clamp 1 further comprises an actuating device 8 (hidden in figure 1) operatively connected with the proximal and distal jaws 5, 6 and with a trigger lever 9 arranged at the handle 3 such that manual actuation of the trigger lever 9 causes the jaws 5, 6 to approximate and, hence, to clamp the prolapsed tissue 10 (Figure 5). The actuating device 8 is also adapted to lock the jaws in their mutual position when the trigger lever is not further moved or held in order to automatically hold the jaws in their tissue clamping configuration. According to an advantageous embodiment, the actuating device is configured to allow the jaws to depart from another (e.g. in response to a backward movement of the trigger lever) in order to release the clamped tissue, even though this feature might not be indispensable in cases in which the excised tissue volume can be removed directly together with the clamping head 2 when it is still clamped.

The not shown actuating device comprises e.g. a pinion and rack gear adapted to transform the rotational movement of the trigger lever 9 in a relative translational movement between the pulling rod 7 and the outer tubular insertion shaft 4 to generate the above described approximation of the jaws 5, 6.

In order to better visualize and access the tissue intended to be treated, the clamping head 2, particularly the proximal jaw 5 defines three proximal access apertures 11 arranged so that they allow the passage of and access to the mucosal tissue associated to the three major internal hemorrhoidal vessels. As can be seen from the figure, the diameter of the insertion shaft 4 is smaller, preferably less than a half or even a third, of the diameter of the clamping head 2, in order that the proximal access apertures 11 are easily transanally visualized or proximally accessed by further instruments without this access being hindered by the insertion shaft 4.

The proximal jaw 5 comprises a slender proximal circular clamping ring 12 or ring-segment which determines a curved tissue clamping line. The proximal clamping ring 12 is connected to the insertion shaft by a proximal spacing frame, particularly by three slender radial spokes 13 arranged at an angular distance of 120° and defining, together with the proximal clamping ring 12, the above said access apertures 11. The spokes 13 are inclined with respect to the longitudinal axis X such that the proximal spacing frame defines in a longitudinal cross-section a triangle having a distal base at the ring 12 and a proximal vertex at the end of the insertion shaft 4. This enables the surgical clamp to assure a well defined clamping line exactly and only along the clamping ring 12 which protrudes distally with respect to proximal spacing frame 13 and the insertion shaft 4. The circumferential thickness of the spokes 13 is much smaller than, preferably less than one third or one fourth, of the circumferential extension of the proximal access apertures 11 defined therebetween.

The distal jaw 6 comprises a distal circular clamping ring 14 or ring-segment complementary to the proximal ring 12 or ring segment and connected by means of a distal spacing frame, particularly by three slender radial spokes 15 arranged at an angular distance of 120°, to the pulling rod 7 received by the insertion shaft 4.

In order to assure a well defined and precise clamping line, the distal spokes 15 are inclined such that the distal clamping ring 14 proximally protrudes with respect to the distal spacing frame.

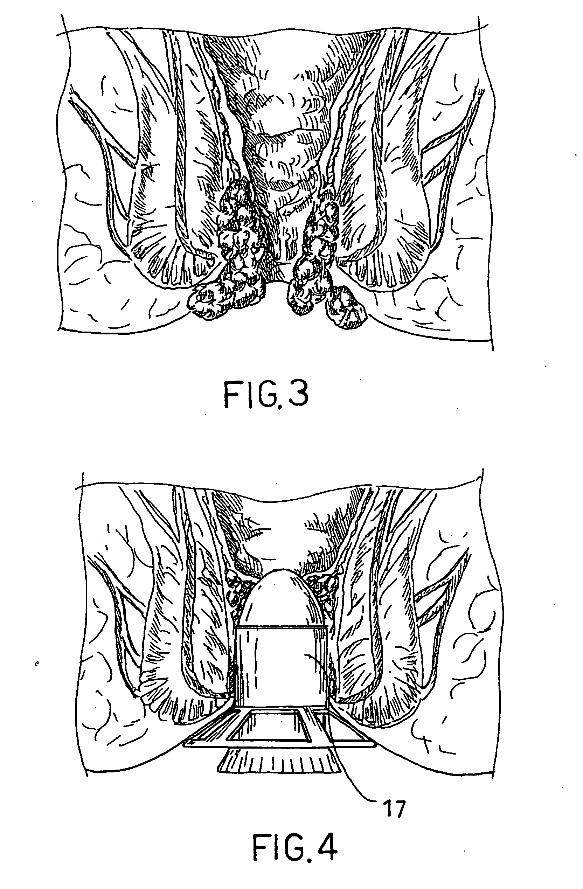

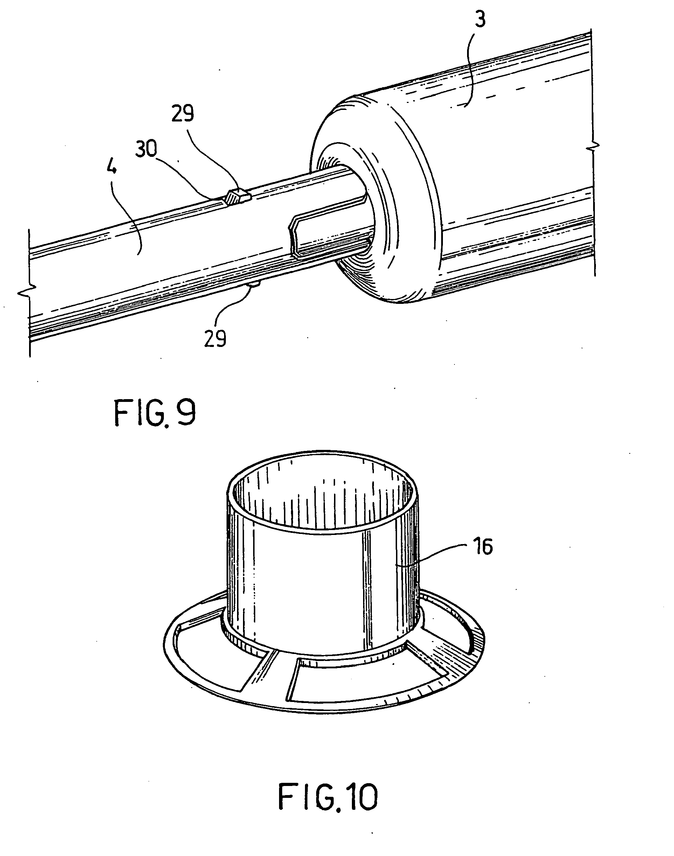

Figure 2 is a proximal end view of the surgical tissue clamp 1 applied to a ring of anal mucosal membrane. From the figure, the skilled person will appreciate that a comparatively good direct visualization and access is provided to both the prolapsed tissue 10 held within the jaws and the ring of tissue 10' radially outside and adjacent to the jaws which are the main areas of interest for the performance of fixation of tissue and removal of excess volume of prolapsed tissue. As can be seen from figure 2 as well as from the subsequent figures 3 to 5, the surgical tissue clamp 1 according to the invention can be inserted after placement of a per se known anal dilator 16 (Fig. 10) and an annular area of tissue 10' intended to be treated is exposed between and confined by the proximal clamping ring 12 and the anal dilator 16.

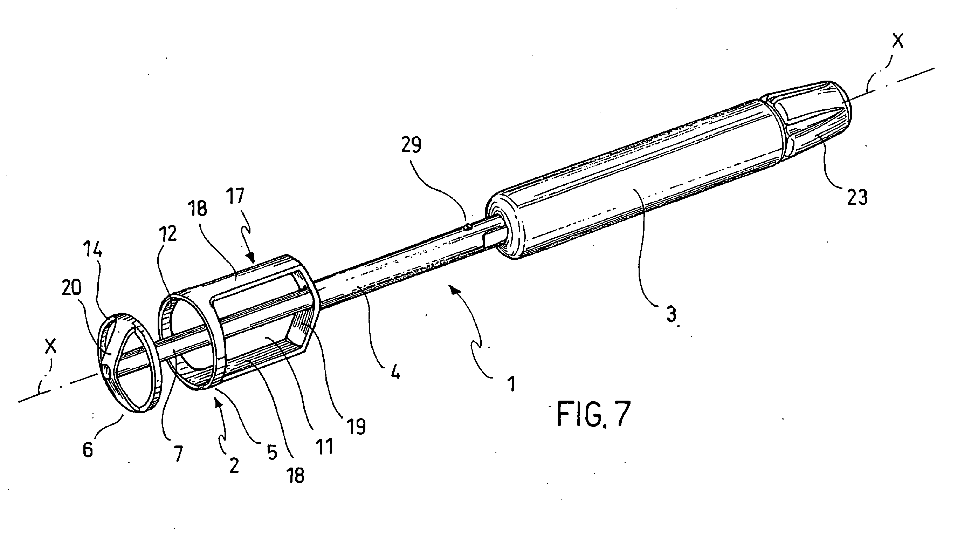

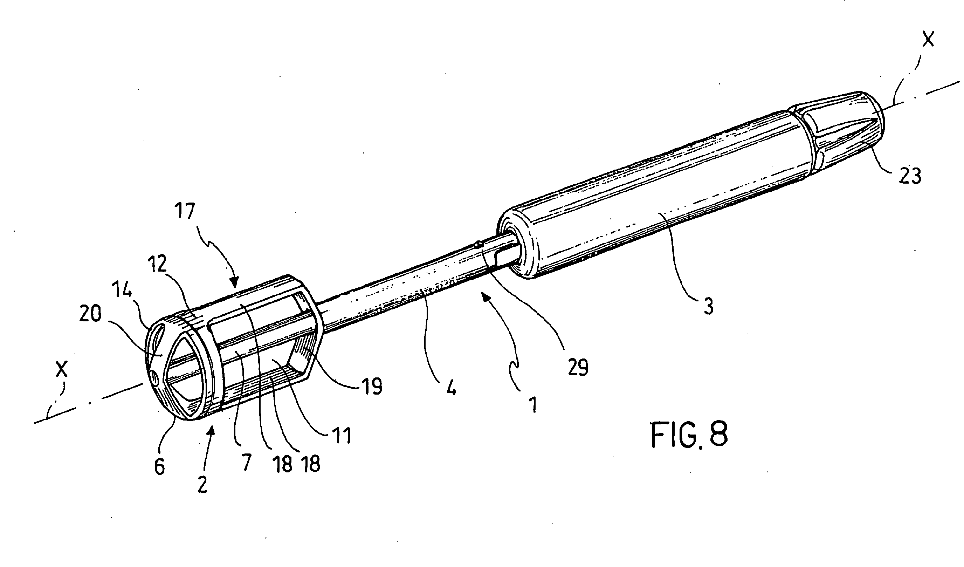

Figures 7 and 8 illustrate a second embodiment of the surgical tissue clamp 1 in an open jaw and closed jaw configuration, respectively, wherein same reference numerals denote same components.

According to this embodiment, the proximal spacing frame comprises a substantially U shaped frame 17 having two longitudinal approximately parallel opposite legs 18 distally connected to the proximal clamping ring 12 and a proximal base spoke 19 connecting the legs 18 to the insertion shaft 4 such that the access apertures 11 defined by the proximal ring 12 and the proximal spacing frame 17 expose the clamped tissue 10, 10' both proximally and laterally (radially).

The distal spacing frame comprises two opposite radial spokes 20 connecting the distal clamping ring 14 to the pulling rod 7. Also in this embodiment, the clamping rings 12, 14 protrude with respect to their spacing frames in order to define an undisturbed clamping line.

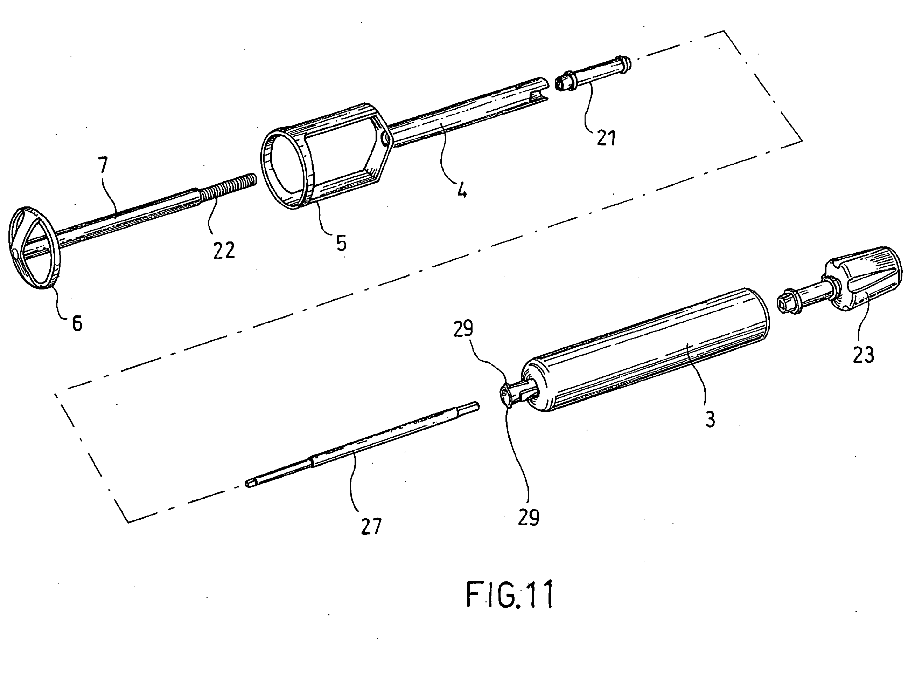

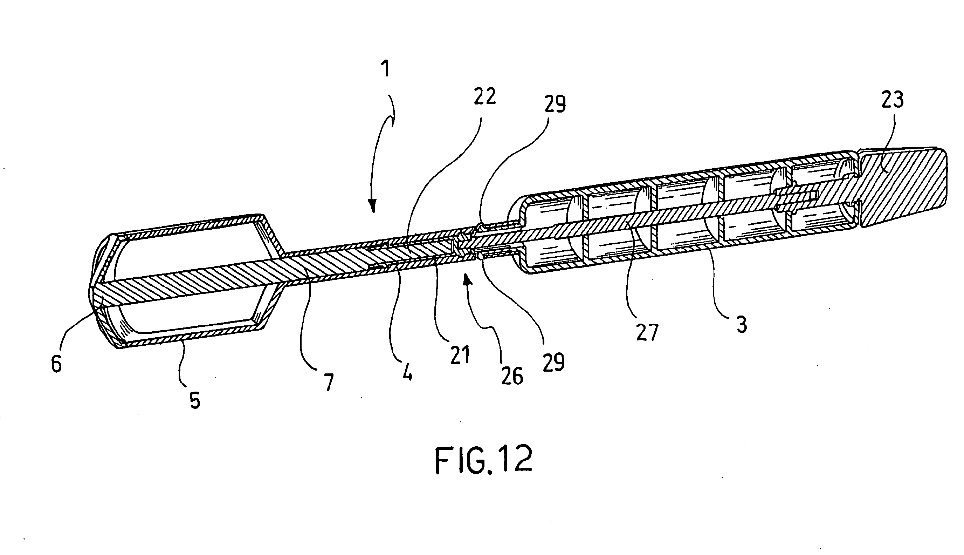

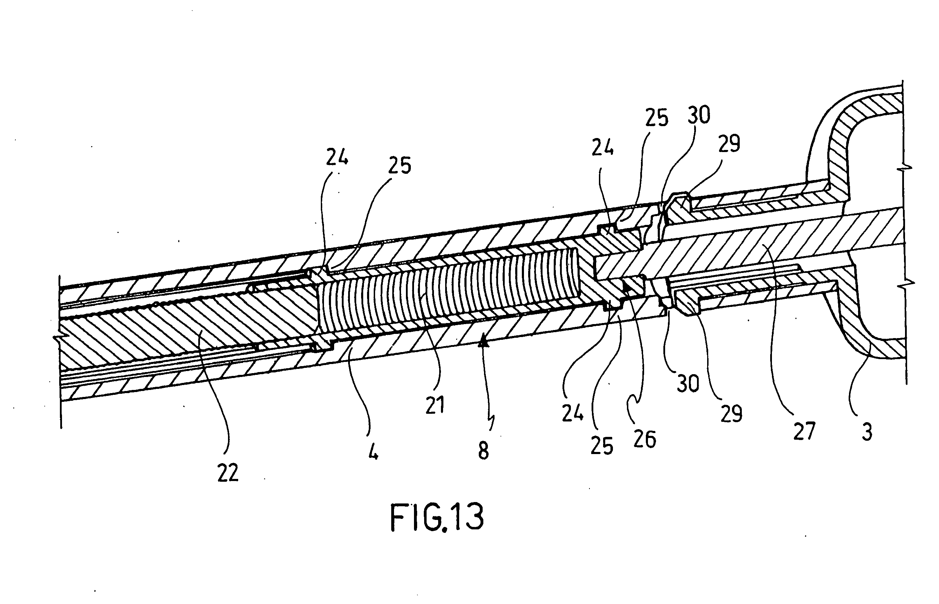

The actuating device 8, which is illustrated in detail in figures 11 to 13 and which could also be implemented in the tissue clamp according to the first embodiment, comprises a jackscrew 21 which is rotatable but translationally locked with respect to the proximal jaw 5, a threaded connecting portion 22 of the pulling rod 7 meshing the jackscrew 21 and a rotating knob 23 mounted on the handle 3 and coupled to rotate with the jackscrew 21 such that a rotation of the rotating knob 23 causes the jackscrew 21 to screw on the pulling rod 7 (which is rotationally constrained so that it cannot rotate together with the jackscrew) thereby approximating or separating the jaws 5, 6.

The jackscrew 21 is preferably housed inside the insertion shaft 4 and comprises an external shape of a body of revolution (with respect to the longitudinal axis of the insertion shaft 4) with two annular flanges 24 engaging corresponding internal annular recesses 25 of the insertion shaft in order to realize the above said rotatable but axially locked support. The jackscrew 21 defines a distally open and internally threaded hole 28 adapted to receive the externally threaded proximal connecting portion 22 of the pulling rod 7 and a preferably polygonal proximal seat 26 for detachably engaging the distal end of a rotary rod 27 having complementary shape, such that the jackscrew 21 can be torsionally coupled and detached from rotary rod 27. Rotary rod 27 extends from jackscrew 21 proximally through the handle 3 and its proximal end is coupled to rotate with the rotating knob 23 which is arranged at the proximal end of handle 3, preferably coaxially to the longitudinal instrument axis X.

The distal end of the handle 3 forms a detachable connecting portion with two opposite elastically supported snapper teeth 29 configured to detachably engage two corresponding recesses 30 formed in the tubular wall of the insertion shaft 4 proximally from the jackscrew 21. In this way, after the prolapsed tissue has been clamped by the instrument, the handle 3 and possibly the proximal portion of the insertion shaft 4 can be detached from the clamping head 2, thereby detaching the distal end of the rotary rod 27 from the jackscrew 21, while at least a distal portion of the insertion shaft housing the jackscrew 21 and the pulling rod 7 remain attached to the clamping head 2. As has been already explained above, the possibility to remove the handle provides a much better visual and instrumental access to the prolapsed tissue during its manipulation in a hemorrhoidopexy or hemorrhoidectomy. Of course, even though the handle detaching feature has been described in detail in connection with the second embodiment, it will be apparent for those skilled in the art that such a feature can be also implemented in the other embodiments of the present invention.

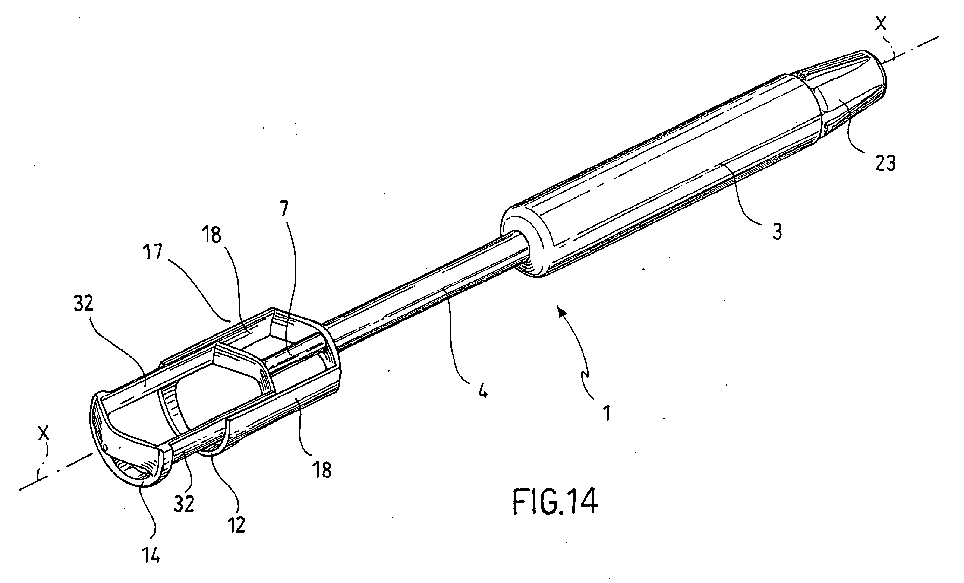

Fig. 14 illustrates a third embodiment of the surgical tissue clamp 1, in which like reference numerals denote like features. According to this embodiment, the proximal and distal jaws 5, 6 comprise slender semicircular proximal and distal clamping ring segments 12, 14 connected to the insertion shaft 4 and to the pulling rod 7, respectively, by an U-shaped proximal spacing frame 17 and an U-shaped or quadrangular distal spacing frame 31. The proximal longitudinal legs 18 of the proximal spacing frame and the distal longitudinal legs 32 of the distal spacing frame 31 are arranged very close to another, preferably overlapping, so that the proximal access apertures 11 defined by the proximal spacing frame 17 are not obstructed by the distal spacing frame.

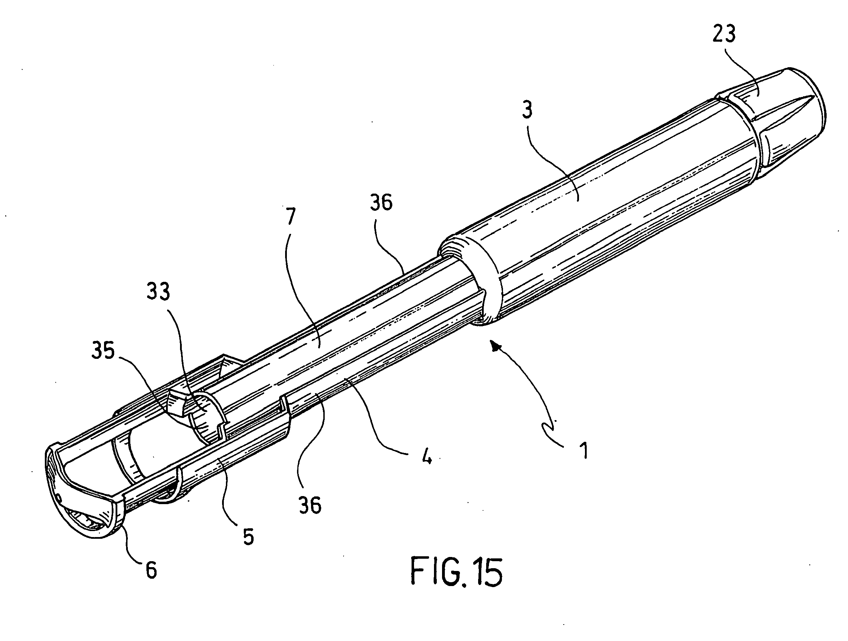

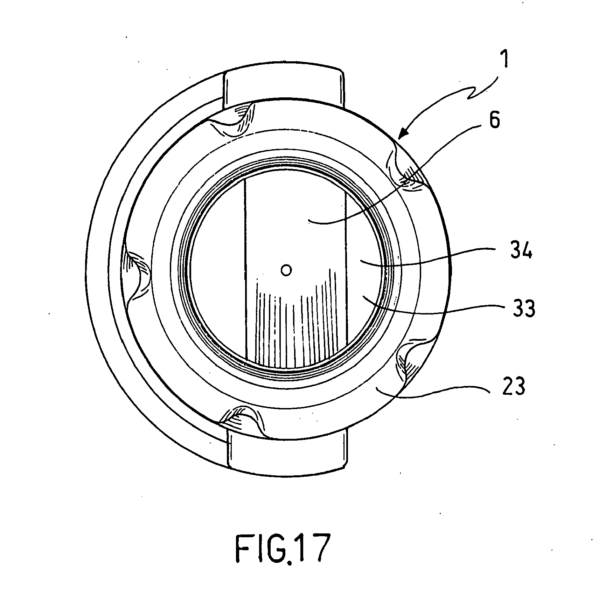

Figures 15 to 17 illustrate a fourth embodiment of the invention, according to which the surgical tissue clamp defines internally a longitudinal scope channel 33 adapted to pass an instrument, particularly a scope to the clamping head 2. the scope channel 33 extends substantially along the longitudinal axis X of the tissue clamp 1 and comprises a proximal inlet opening 34 formed in the handle 3 and a distal outlet opening 35 formed in the insertion shaft 4 proximally of the proximal and distal jaws 5, 6 and directly facing towards or through the proximal access aperture/s 11.

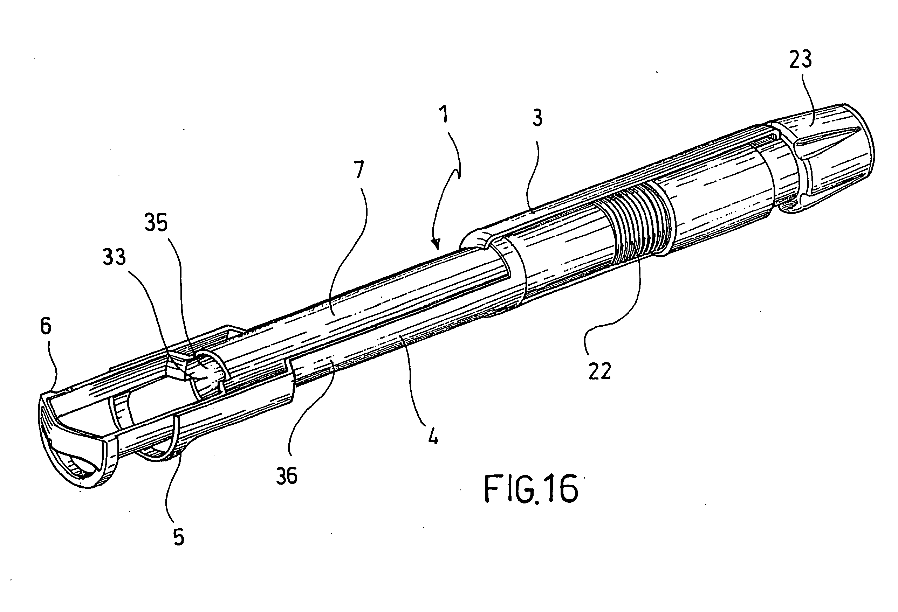

In order to conveniently embody the scope channel 33, both handle 3 and actuation device 8 are fabricated of hollow tubular members defining internally the scope channel 33. With particular reference to figure 16, the pulling rod 7 is embodied as a hollow tubular member received between two opposite cylinder segments 36 defining the insertion shaft 4. The proximal end of the hollow pulling rod 7 is externally threaded and engages a corresponding internal thread directly formed at the inside of the hollow tubular rotating knob 23 which embodies both the manual manipulation member and the jackscrew member. Even though in the exemplary embodiment illustrated in figures 18 and 19, the handle is not detachable from the insertion shaft, such a detachable connection can be advantageously applied also to this embodiment.

With reference to figures 3 to 6, a hemorrhoidopexy or hemorrhoidectomy is advantageously performed by inserting the anal dilator into the anal canal in order to provide access to the operational site. Then, the surgical tissue clamp 1 is inserted through the anal dilator in the anal canal and the prolapsed tissue is pulled between the proximal and distal jaws and if necessary through the proximal access aperture of the instrument. To this end, a purse string can be applied to the prolapsed tissue and the proximal end of the suture is pulled through the proximal access aperture or, alternatively, other suitable grasping and pulling instruments, such as surgical graspers, hemostats, etc. can be used to place the tissue between the jaws of the clamp 1, while the prolapsed tissue "inside" the jaws is visible through the proximal access apertures. After correct positioning of the tissue to be clamped, the rotating knob 23 or the trigger lever 9 are manually moved to approximate the jaws 5, 6 and clamp the tissue along a precise arch-shaped or ring shaped clamping line and secure the tissue for the remainder of the procedure which preferably involves fixation of tissue and removal of excess tissue volume by the most appropriate procedure available to the surgeon. It should be noted that the appropriateness of the specific procedure can depend from the specific skills and preferences of the surgeon, from the specific pathologic and anatomic situation of the patient as well as from the cost of the medical devices (this can become very important in the poorer regions of the planet where many hospitals cannot afford expensive instrumentation). Exemplary choices of surgical devices for fixating the prolapsed mucosa are among others surgical suture, surgical staples, surgical adhesives and harmonic or ultrasonic devices, all preferably distinct and not incorporated in the tissue clamp 1. Exemplary choices of surgical devices for removing the excess tissue volume are among others surgical knifes or scalpels, scissors, harmonic or ultrasonic devices and radiofrequency devices, all preferably distinct and not incorporated in the tissue clamp 1. As will be immediately appreciated by those skilled in the art, the above described method and device provide a very versatile and cost-effective alternative to the all-in-one-device-approach of the

### prior art.

While the present invention has been illustrated by description of several embodiments and while the illustrative embodiments have been described in considerable detail, it is not the intention to restrict or in any way limit the scope of the appended claims to such detail. Additional advantages and modifications may readily appear to those skilled in the art.

## Claims

1. A surgical tissue clamp (1), particularly for holding prolapsed tissue (10, 10') during a hemorrhoidopexy or hemorrhoidectomy, comprising:
- an elongate insertion shaft (4),
- a handle (3) arranged at a proximal end of the insertion shaft (4);
- a clamping head (2) arranged at a distal end of the insertion shaft (4) and including a proximal jaw (5) and a distal jaw (6) movable relative to the proximal jaw (5) and configured to cooperate with the proximal jaw (5) for clamping said prolapsed tissue (10) in a substantially radial orientation with respect to a longitudinal axis (X) of the clamping head (2);
- an actuating device (8) operatively connected with said proximal and distal jaws (5, 6) and adapted to approximate said jaws and to hold said jaws in a tissue clamping configuration,
in which said clamping head (2) defines at least one proximal access aperture (11) configured to expose the clamped tissue proximally, in which the proximal jaw (5) comprises a proximal clamping ring (12) or ring-segment determining a tissue clamping line and being connected to the insertion shaft (4) by a proximal_spacing frame (13), said spacing frame (13) and said ring (12) or ring-segment defining said proximal access apertures (11),
**characterized in that** said proximal spacing frame comprises slender radial spokes (13) and the circumferential thickness of said spokes (13) is smaller than the circumferential extension of the proximal access apertures (11) defined therebetween.

2. A surgical tissue clamp (1) according to claim 1, wherein:
- the distal jaw (6) comprises a distal clamping ring (14) or ring-segment complementary to the proximal ring (12) or ring segment of the proximal jaw (5) and connected by means of a distal spacing frame (15) to a pulling rod (7) received by the insertion shaft (4),

3. A surgical tissue clamp (1) according to claim 1 or 2, wherein said proximal spacing frame comprises a substantially U shaped frame (17) having two longitudinal approximately parallel legs (18) distally connected to the proximal clamping ring (12) or ring-segment and a proximal base spoke (19) connecting said U-shaped frame (17) to the insertion shaft (4) such that the access apertures (11) defined by the proximal clamping ring (12) or ring-segment and the proximal spacing frame expose the clamped tissue (10) both proximally and laterally.

4. A surgical tissue clamp (1) according to any one of claims 2 to 3, wherein said distal spacing frame comprises radial spokes connecting the distal clamping ring (14) or ring-segment to the pulling rod (4).

5. A surgical tissue clamp (1) according to claim 3, wherein said distal spacing frame comprises a substantially U-shaped or quadrangular frame (31) having two longitudinal approximately parallel legs (32) distally connected to the distal clamping ring (14) or ring-segment of the distal jaw (6) and a proximal base spoke connecting said U-shaped or quadrangular frame (31) to the pulling rod (7) received in said insertion shaft (4), said distal spacing frame (31) being configured such that its longitudinal legs (32) are close to, preferably overlapping the longitudinal legs (18) of the proximal spacing frame (17).

6. A surgical tissue clamp (1) according to any one of the preceding claims, wherein said insertion shaft (4) is rigid and straight or curved.

7. A surgical tissue clamp (1) according to any one of the preceding claims,
wherein said actuating device (8) comprises:
- a manual actuating member (9; 23);
- a moving mechanism (21, 22) operatively connected to the manual actuating member (9; 23) and to the proximal and distal jaws (5, 6) and adapted to approximate and separate the jaws (5, 6) in response to an actuating movement of said manual actuating member (9; 23),
- a locking mechanism (21, 22) operatively connected to the proximal and distal jaws (5, 6) and configured to lock the jaws (5, 6) in said tissue clamping configuration.

8. A surgical tissue clamp (1) according to the preceding claim, wherein said locking mechanism (21, 22) is arranged in said insertion shaft (4), and said handle (3) is detachable from said insertion shaft (4) on the proximal side of said locking mechanism (21, 22), while the locking mechanism (21, 22) keeps the jaws (5, 6) locked in their tissue clamping configuration.

9. A surgical tissue clamp (1) according to claim 6 or 7, wherein said actuating device (8) comprises:
- a jackscrew (21) which is rotatable but translationally locked with respect to the proximal jaw (5) ;
- a threaded connecting portion (22) of the pulling rod (7) of the distal jaw (6) meshing said jackscrew (21);
- a rotating knob (23) mounted on the handle (3) and torsionally coupled with the jackscrew (21) such that a rotation of the rotating knob (23) causes the jackscrew (21) to screw on the pulling rod (7) thereby approximating or separating the jaws (5, 6),
wherein the frictional resistance in said screw connection between the jackscrew (21) and the pulling rod (7) provides said jaw locking mechanism.

10. A surgical tissue clamp (1) according to any one of the preceding claims, defining internally a scope channel (33) adapted to pass a scope through the tissue clamp (1) up to the clamping head (2), said scope channel (33) having a proximal inlet opening (34) formed in said handle (3) and a distal outlet opening (35) formed in said insertion shaft (4) proximally of said clamping head (2) and directly facing towards or through said proximal access aperture/s (11).

11. A surgical tissue clamp (1) according to the preceding claim, wherein said actuation device is made of hollow tubular members defining internally said scope channel (33).

## Patentansprüche

1. Chirurgische Gewebeklemmvorrichtung (1), insbesondere zum Halten von prolabiertem Gewebe (10,10') während einer Hämorrhoidopexie oder einer Hämorrhoidektomie, umfassend:
- einen langestreckten Einführungsschaft (4);
- einen Griff (3), der an einem proximalen Ende des Einführungsschafts (4) angeordnet ist;
- einen Klemmkopf (2), der an einem distalen Ende des Einführungsschafts (4) angeordnet ist und der eine proximale Backe (5) und eine distale Backe (6) umfasst, die relativ zur proximalen Backe (5) bewegbar ist und konfiguriert ist, mit der proximalen Backe (5) zusammen zu wirken, um das prolabierte Gewebe (10) in einer im Wesentlichen radialen Orientierung bezüglich einer Längsachse (X) des Klemmkopfs (2) einzuklemmen;
- eine Betätigungsvorrichtung (8), die mit der proximalen und der distalen Backe (5,6) betriebsmäßig verbunden ist und angepasst ist, die Backen anzunähern und die Backen in einer Gewebeklemmkonfiguration zu halten, in welcher der Klemmkopf (2) wenigstens eine proximale Zugangsöffnung (11) definiert, die konfiguriert ist, das geklemmte Gewebe proximal freizulegen, wobei die proximale Backe (5) einen proximalen Klemmring (12) oder ein Ringsegment umfasst, welcher/welches eine Gewebeklemmlinie festlegt und welcher/welches mit dem Einführungsschaft (4) mittels eines proximalen Abstand-Rahmens (13) verbunden ist, wobei der Abstand-Rahmen (13) und der Ring (12) oder das Ringsegment die proximalen Zugangsöffnungen (11) definieren,
**dadurch gekennzeichnet,**
**dass** der proximale Abstand-Rahmen schmale radiale Speichen (13) umfasst,
und **dass** die Umfangsdicke der Speichen (13) kleiner als die Umfangsausdehnung der dazwischen definierten proximalen Zugangsöffnungen (11) ist.

2. Chirurgische Gewebeklemmvorrichtung (1) nach Anspruch 1,
wobei die distale Backe (6) einen distalen Klemmring (14) oder ein Ringsegment umfasst, welcher/welches zum proximalen Ring (12) oder Ringsegment der proximalen Backe (5) komplementär ist und mittels eines distalen Abstand-Rahmens (15) mit einer in dem Einführungsschaft (4) aufgenommenen Zugstange (7) verbunden ist.

3. Chirurgische Gewebeklemmvorrichtung (1) nach Anspruch 1 oder 2,
wobei der proximale Abstand-Rahmen einen im Wesentlichen U-förmigen Rahmen (17) umfasst, welcher zwei längliche in etwa parallele Beine (18) aufweist, die distal mit dem proximalen Klemmring (12) oder Ringsegment verbunden sind und eine proximale Basisspeiche (19) aufweist, die den U-förmigen Rahmen (17) mit dem Einführungsschaft (4) derart verbindet, dass die durch den proximalen Klemmring (12) oder das Ringsegment definierten Zugangsöffnungen (11) und der proximale Abstand-Rahmen das geklemmte Gewebe (10) sowohl proximal als auch lateral freigeben.

4. Chirurgische Gewebeklemmvorrichtung (1) nach einem der Ansprüche 2 bis 3,
wobei der distale Abstand-Rahmen radiale Speichen umfasst, die den distalen Klemmring (14) oder das Ringsegment mit der Zugstange (4) verbinden.

5. Chirurgische Gewebeklemmvorrichtung (1) nach Anspruch 3,
wobei der distale Abstand-Rahmen einen im Wesentlichen U-förmigen oder viereckigen Rahmen (31) umfasst, welcher zwei längliche in etwa parallele Beine (32) aufweist, die distal mit dem distalen Klemmring (14) oder Ringsegment der distalen Backe (6) verbunden sind und eine proximale Basispeiche umfasst, die den U-förmigen oder viereckigen Rahmen (31) mit der in dem Einführungsschaft (4) aufgenommenen Zugstange (7) verbindet,
wobei der distale Abstand-Rahmen (31) derart konfiguriert ist, dass seine länglichen Beine (32) sich nahe an, vorzugsweise überlappend mit, den länglichen Beinen (18) des proximalen Abstand-Rahmens (17) befinden.

6. Chirurgische Gewebeklemmvorrichtung (1) nach einem der vorhergehenden Ansprüche,
wobei der Einführungsschaft (4) steif und gerade oder gebogen ist.

7. Chirurgische Gewebeklemmvorrichtung (1) nach einem der vorhergehenden Ansprüche,
wobei die Betätigungsvorrichtung (8) umfasst:
- ein manuelles Betätigungselement (9; 23);
- eine Bewegungsvorrichtung (21, 22), die mit dem manuellen Betätigungselement (9; 23) und mit den proximalen und den distalen Backen (5, 6) betriebsmäßig verbunden ist und angepasst ist, die Backen (5, 6) als Antwort auf eine Betätigungsbewegung des manuellen Betätigungselements (9; 23) anzunähern und zu trennen,
- eine Verriegelungsvorrichtung (21, 22), die mit den proximalen und distalen Backen (5, 6) betriebsmäßig verbunden ist und konfiguriert ist, die Backen (5, 6) in der Gewebeklemmkonfiguration zu verriegeln.

8. Chirurgische Gewebeklemmvorrichtung (1) nach dem vorhergehenden Anspruch,
wobei die Verriegelungsvorrichtung (21, 22) in dem Einführungsschaft (4) angeordnet ist und der Griff (3) von dem Einführungsschaft (4) auf der proximalen Seite der Verriegelungsvorrichtung (21, 22) abnehmbar ist, während die Verriegelungsvorrichtung (21, 22) die Backen (5, 6) in ihrer Gewebeklemmkonfiguration verriegelt hält.

9. Chirurgische Gewebeklemmvorrichtung (1) nach Anspruch 6 oder 7,
wobei die Betätigungsvorrichtung (8) umfasst:
- eine Druckschraube (21), welche drehbar aber translatorisch verriegelt bezüglich der proximalen Backe (5) ist;
- einen mit einem Gewinde versehener Verbindungsabschnitt (22) der Zugstange (7) der distalen Backe (6), welcher mit der Druckschraube (21) im Eingriff steht;
- einen Drehknopf (23), der auf dem Griff (3) montiert ist und mit der Druckschraube (21) derart torsionsmäßig gekoppelt ist, dass eine Drehung des Drehknopfs (23) die Druckschraube (21) veranlasst, sich auf die Zugstange (7) anzuschrauben, wobei dadurch die Backen (5, 6) angenähert oder getrennt werden,
wobei der Reibungswiderstand in der Schraubenverbindung zwischen der Druckschraube (21) und der Zugstange (7) die Backen-Verriegelungsvorrichtung bereitstellt.

10. Chirurgische Gewebeklemmvorrichtung (1) nach einem der vorhergehenden Ansprüche,
welche in dem Inneren einen Instrumentenkanal (33) definiert, welcher angepasst ist, um durch die Gewebeklemme (1) zum Klemmkopf (2) hin ein Instrument zu führen,
wobei der Instrumentenkanal (33) eine in dem Griff (3) gebildete proximale Eintrittsöffnung (34) und eine distale Austrittsöffnung (35) aufweist, die in dem Einführungsschaft (4) proximal zu dem Klemmkopf (2) gebildet ist und direkt auf oder durch die proximale Zugangsöffnung / proximalen Zugangsöffnungen (11) weist.

11. Chirurgische Gewebeklemmvorrichtung (1) nach dem vorhergehenden Anspruch,
wobei die Betätigungsvorrichtung aus hohlen, röhrenförmigen Elementen, die im Inneren den Instrumentenkanal (33) definieren, gebildet ist.

## Revendications

1. Pince chirurgicale pour tissus (1), en particulier pour tenir des tissus en prolapsus (10, 10') pendant une hémorroïdectomie ou une hémorroïdopexie, comprenant :
- une tige d'introduction allongée (4),
- une poignée (3) agencée à une extrémité proximale de la tige d'introduction (4) ;
- une tête de pince (2) agencée à une extrémité distale de la tige d'introduction (4) et incluant une mâchoire proximale (5) et une mâchoire distale (6) mobile par rapport à la mâchoire proximale (5) et configurée pour coopérer avec la mâchoire proximale (5) pour pincer lesdits tissus en prolapsus (10) dans une orientation sensiblement radiale par rapport à un axe longitudinal (X) de la tête de pince (2) ;
- un dispositif d'actionnement (8) fonctionnellement connecté à ladite mâchoire proximale et ladite mâchoire distale (5, 6) et adapté à approcher lesdites mâchoires et à tenir lesdites mâchoires dans une configuration de pincement de tissus,
dans laquelle ladite de tête de pince (2) définit au moins une ouverture d'accès proximale (11) configurée pour exposer les tissus pincés en direction proximale,
dans laquelle la mâchoire proximale (5) comprend une bague de pince proximale (12) ou un segment annulaire déterminant une ligne de pincement de tissus et étant connectée à la tige d'introduction (4) par un cadre d'espacement proximal (13), ledit cadre d'espacement (13) et ladite bague (12) ou ledit segment annulaire définissant lesdites ouvertures d'accès proximales (11),
**caractérisée en ce que** ledit cadre d'espacement proximal comprend des rayons radiaux minces (13) et l'épaisseur circonférentielle desdits rayons (13) est inférieure à l'extension circonférentielle des ouvertures d'accès proximales (11) définies entre eux.

2. Pince chirurgicale pour tissus (1) selon la revendication 1, dans laquelle :
- la mâchoire distale (6) comprend une bague de pince distale (14) ou un segment annulaire complémentaire de la bague proximale (12) ou du segment annulaire de la mâchoire proximale (5) et connectée au moyen d'un cadre d'espacement distal (15) à une tige de traction (7) reçue par la tige d'introduction (4).

3. Pince chirurgicale pour tissus (1) selon la revendication 1 ou 2, dans laquelle ledit cadre d'espacement proximal comprend un cadre sensiblement en forme de U (17) ayant deux pieds longitudinaux approximativement parallèles (18) connectés en situation distale à la bague de pince proximale (12) ou au segment annulaire, et un rayon de base proximal (19) qui connecte ledit cadre en forme de U (17) à la tige d'introduction (4) de telle manière que les ouvertures d'accès (11) définies par la bague de pince proximale (12) ou le segment annulaire, et le cadre d'espacement proximal, exposent les tissus pincés (10) à la fois en direction proximale et en direction latérale.

4. Pince chirurgicale pour tissus (1) selon l'une quelconque des revendications 2 à 3, dans laquelle ledit cadre d'espacement distal comprend des rayons radiaux qui connectent la bague de pince distale (14) ou le segment annulaire à la tige de traction (4).

5. Pince chirurgicale pour tissus (1) selon la revendication 3, dans laquelle ledit cadre d'espacement distal comprend un cadre sensiblement en forme de U ou un cadre quadrangulaire (31) ayant deux pieds longitudinaux approximativement parallèles (32) connectés de manière distale à la bague de pince distale (14) ou au segment annulaire de la mâchoire distale (6) et un rayon de base proximal qui connecte ledit cadre en forme de U ou cadre quadrangulaire (31) à la tige de traction (7) reçue dans ladite tige d'introduction (4), ledit cadre d'espacement distal (31) étant configuré de telle façon que ses pieds longitudinaux (32) sont proches des pieds longitudinaux (18), et de préférence en chevauchement avec ces derniers, du cadre d'espacement proximal (17).

6. Pince chirurgicale pour tissus (1) selon l'une quelconque des revendications précédentes, dans laquelle ladite tige d'introduction (4) est rigide et rectiligne ou incurvée.

7. Pince chirurgicale pour tissus (1) selon l'une quelconque des revendications précédentes,
dans laquelle ledit dispositif d'actionnement (8) comprend :
- un élément d'actionnement manuel (9 ; 23) ;
- un mécanisme de mouvement (21, 22) fonctionnellement connecté à l'élément d'actionnement manuel (9 ; 23) et à la mâchoire proximale et la mâchoire distale (5, 6), et adapté à approcher et à séparer les mâchoires (5, 6) en réponse à un mouvement d'actionnement dudit élément d'actionnement manuel (9 ; 23),
- un mécanisme de blocage (21, 22) fonctionnellement connecté à la mâchoire proximale et à la mâchoire distale (5, 6) et configuré pour bloquer les mâchoires (5, 6) dans ladite configuration de pincement de tissus.

8. Pince chirurgicale pour tissus (1) selon la revendication précédente, dans laquelle ledit mécanisme de blocage (21, 22) est agencé dans ladite tige d'introduction (4), et ladite poignée (3) est détachable vis-à-vis de ladite tige d'introduction (4) sur le côté proximal dudit mécanisme de blocage (21, 22), alors que le mécanisme de blocage (21, 22) maintient les mâchoires (5, 6) bloquées dans leur configuration de pincement de tissus.

9. Pince chirurgicale pour tissus (1) selon la revendication 6 ou 7, dans lequel ledit dispositif d'actionnement (8) comprend :
- une vis-vérin (21) qui est capable de tourner mais qui est bloquée en translation par rapport à la mâchoire proximale (5) ;
- une portion de connexion à pas de vis (22) de la tige de traction (7) de la mâchoire distale (6) en engagement vissé avec ladite vis-vérin (21) ;
- un bouton rotatif (23) monté sur la poignée (3) et couplé en torsion avec la vis-vérin (21) de telle façon qu'une rotation du bouton rotatif (23) amène la vis-vérin (21) à se visser dans la tige de traction (7) en approchant ou en séparant ainsi les mâchoires (5, 6),
dans laquelle la résistance de friction dans ladite connexion vissée entre la vis-vérins (21) et la tige de traction (7) constitue ledit mécanisme de blocage de mâchoire.

10. Pince chirurgicale pour tissus (1) selon l'une quelconque des revendications précédentes, définissant à l'intérieur un canal d'observation (33) adapté à laisser passer un endoscope à travers la pince pour tissus (1) jusqu'à la tête de pince (2), ledit canal d'observation (33) ayant une ouverture d'entrée proximale (34) formée dans ladite poignée (3) et une ouverture de sortie distale (35) formée dans ladite tige d'introduction (4) en position proximale par rapport à ladite tête de pince (2) et directement tournée vers ou à travers ladite/lesdites ouverture(s) d'accès proximale(s) (11).

11. Pince chirurgicale pour tissus (1) selon la revendication précédente, dans laquelle ledit dispositif d'actionnement est constitué d'éléments tubulaires creux définissant à l'intérieur ledit canal d'observation (33).
